(19) Europäisches Patentamt
European Patent Office
Office européen des brevets

(11) EP 3 199 559 B1

(12) EUROPEAN PATENT SPECIFICATION

(45) Date of publication and mention of the grant of the patent:
**04.09.2019 Bulletin 2019/36**

(21) Application number: **15845297.9**

(22) Date of filing: **24.09.2015**

(51) Int Cl.:
***C08F 20/36*** *(2006.01)* ***C07C 235/08*** *(2006.01)*
***C08F 12/26*** *(2006.01)* ***C08F 26/02*** *(2006.01)*

(86) International application number:
**PCT/JP2015/076976**

(87) International publication number:
**WO 2016/047705 (31.03.2016 Gazette 2016/13)**

(54) **VINYL MONOMER HAVING DIGLYCOLAMIDIC-ACID-TYPE LIGAND**

VINYLMONOMER MIT LIGAND VOM DIGLYCOLAMIDSÄURETYP

MONOMÈRE DE VINYLE AYANT UN LIGAND DE TYPE ACIDE DIGLYCOLAMIDIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.09.2014 JP 2014194203**

(43) Date of publication of application:
**02.08.2017 Bulletin 2017/31**

(73) Proprietor: **Nitto Denko Corporation**
**Ibaraki-shi, Osaka 567-8680 (JP)**

(72) Inventors:
- **MURAKI, Yuzo**
  **Ibaraki-shi**
  **Osaka 567-8680 (JP)**
- **HIGUCHI, Hiroyuki**
  **Ibaraki-shi**
  **Osaka 567-8680 (JP)**
- **NISHIGAWARA, Masaya**
  **Ibaraki-shi**
  **Osaka 567-8680 (JP)**
- **OGATA, Takeshi**
  **Tsukuba-shi**
  **Ibaraki 3058569 (JP)**
- **NARITA, Hirokazu**
  **Tsukuba-shi**
  **Ibaraki 3058569 (JP)**
- **TANAKA, Mikiya**
  **Tsukuba-shi**
  **Ibaraki 3058569 (JP)**

(74) Representative: **Müller-Boré & Partner**
**Patentanwälte PartG mbB**
**Friedenheimer Brücke 21**
**80639 München (DE)**

(56) References cited:
**EP-A1- 2 592 068**
**WO-A1-2014/157225**
**WO-A1-2015/137451**
**JP-A- 2007 327 085**
**JP-A- 2011 504 964**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 199 559 B1

## Description

TECHNICAL FIELD

The present invention relates to a vinyl monomer having a ligand having a diglycolamic acid skeleton (hereinafter, also referred to as a "diglycolamic-acid-type ligand"), which is useful for producing an adsorption separation material for separating and recovering rare earth elements.

BACKGROUND ART

Rare earth elements are essential materials necessary for improving performance of an electronic product such as a storage battery, a light emitting diode or a magnet, and are used in a wide range of industrial fields. With respect to the current supply of rare earth elements, a crisis of these resources has been highlighted because the producing countries are almost limited, the price lacks stability, and the demand is expected to surpass the supply in the near future. For these reasons, many attempts have been made to reduce the amount of rare earth element used and to develop a replacement. At the same time, it is also required to recover rare earth elements from natural mineral resources of low grade, or to regenerate (recycle) rare earth elements from in-process scraps produced during manufacture of products or from wastes such as electronic and electric appliances collected from cities.

As to the recovery process of rare earth elements from natural mineral resources of low grade or the wastes, there is a method for selectively separating and recovering dilute rare earth element ions from an- aqueous solution containing ions of metals, for example, base metals in a high concentration.

As the method for separating and recovering rare earth element ions, a solvent extraction method using an extracting agent or the like (liquid-liquid extraction method), a column extraction method using an ion exchange resin or the like (solid-liquid extraction method) and the like are known. The solvent extraction method is a method in which an aqueous phase containing an aqueous solution containing metal element to be separated is brought into contact with an organic phase containing an extracting agent for extracting the specific metal element and an organic solvent for diluting the extracting agent, to extract the specific metal element with the extracting agent, thereby separating the specific metal element.

In the solvent extraction method or the column extraction method, an adsorbent excellent in adsorption ability for rare earth element ions is used and the rare earth element ions are selectively adsorbed.

It is known that a diglycolamic acid (DGAA) skeleton containing a carboxylic group ($>N-CO-CH_2-O-CH_2-COOH$, $-NH-CO-CH_2-O-CH_2-COOH$) is effective as a multidentate ligand for the adsorption of rare earth element ions, and a solvent extraction method using an extracting agent and column extraction method using a particulate adsorbent, having the skeleton have been studied. As to the solvent extraction method, for example, a technique for extracting a rare earth element by using a solution in which diglycolamic acid represented by the specific chemical formula is dissolved as an extracting agent is proposed (e.g., Patent Document 1). Furthermore, as to the column extraction method, for example, a technique for separating a rare earth element by column adsorption using an adsorbent in which the specific diglycolamide type ligand is introduced into silica gel surface is proposed (e.g., Non-Patent Document 1).

PRIOR ART DOCUMENT

PATENT DOCUMENT

Patent Document 1: JP-A-2007-327085

NON-PATENT DOCUMENT

Non-Patent Document 1: Tsuyoshi Ogata and Mikiya Tanaka, "Separation and recovery of rare earth element ions by novel adsorbent", Rare Earth, The Rare Earth Society of Japan, May 2013, No. 62, pages 178-179

SUMMARY OF THE INVENTION

PROBLEMS THAT THE INVENTION IS TO SOLVE

The suitable extraction method is determined depending on the diglycolamic-acid-type ligand to be used, and the molecular design of a monomer having the diglycolamic-acid-type ligand is an important factor for the separation and adsorption efficiency of rare earth elements

Therefore, an object of the present invention is to provide a novel vinyl monomer having a diglycolamic-acid-type ligand, which is useful for producing an adsorption separation material for separating and recovering rare earth elements.

MEANS FOR SOLVING THE PROBLEMS

The problem described above can be solved by the present invention described below. Specifically, the present invention includes (1) to (5) shown below.

(1) A vinyl monomer having a diglycolamic-acid-type ligand represented by general formula (1) shown below:

[Chem. 1]

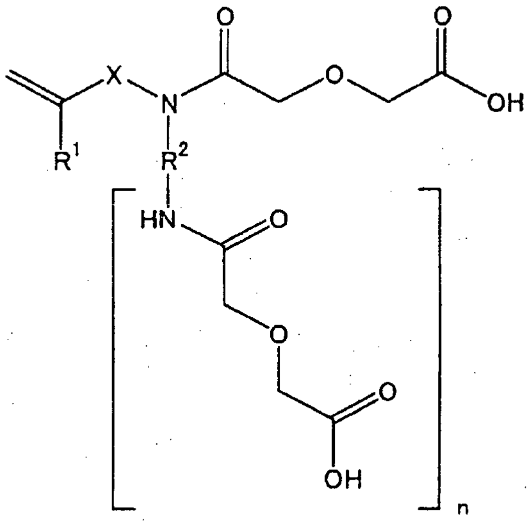

(1)

(in the formula (1), $R^1$ represents a hydrogen atom or an alkyl group having a carbon number of from 1 to 3, when $R^1$ is a hydrogen atom, X represents a single bond or a divalent organic group having a carbon number of from 2 to 7, which may have a substituent, and when $R^1$ is an alkyl group having a carbon number of from 1 to 3, X represents a single bond or a divalent organic group having a carbon number of from 1 to 7, which may have a substituent. n represents 0 or 1, when n is 0, $R^2$ represents a hydrogen atom, an alkyl group having a carbon number of from 1 to 3, an acyl group having a carbon number of from 1 to 2, or an aldehyde group, and when n is 1, $R^2$ represents an alkylene group having a carbon number of from 1 to 3.)

(2) The vinyl monomer as described in (1) above, which is at least one compound selected from the group consisting of a (meth)acrylic acid compound, a (meth)acrylamide compound, a styrene compound, and a vinylamide(imide) compound.

(3) The vinyl monomer as described in (1) or (2) above, which is used for production of an adsorption separation material for a rare earth element.

(4) A (co)polymer containing the vinyl monomer as described in (1) or (2) above.

(5) The (co)polymer as described in (4) above, which is used as an adsorption separation material for a rare earth element.

ADVANTAGE OF THE INVENTION

The vinyl monomer of the present invention is a novel monomer having a diglycolamic-acid-type ligand represented by general formula (1), and according to the adsorption separation material using the vinyl monomer, a rare earth element can be selectively separated and recovered from natural mineral resources of low grade and wastes.

BRIEF DESCRIPTION OF THE DRAWING

FIG. 1 is a graph showing the adsorption amounts of metal ions at each pH of the adsorption separation material.

MODE FOR CARRYING OUT THE INVENTION

The present invention will be described in detail hereinafter.

The vinyl monomer of the present invention is a vinyl monomer having a diglycolamic-acid-type ligand, represented by general formula (1) shown below (hereinafter, also simply referred to as a "vinyl monomer of the present invention").

[Chem. 2]

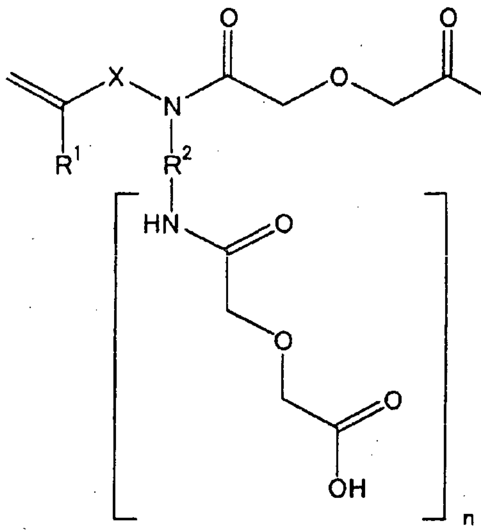

(1)

(In the formula (1), $R^1$ represents a hydrogen atom or an alkyl group having a carbon number of from 1 to 3, when $R^1$ is a hydrogen atom, X represents a single bond or a divalent organic group having a carbon number of from 2 to 7, which may have a substituent, and when $R^1$ is an alkyl group having a carbon number of from 1 to 3, X represents a single bond or a divalent organic group having a carbon number of from 1 to 7, which may have a substituent. n represents 0 or 1, when n is 0, $R^2$ represents a hydrogen atom, an alkyl group having a carbon number of from 1 to 3, an acyl group having a carbon number of from 1 to 2, or an aldehyde group, and when n is 1, $R^2$ represents an alkylene group having a carbon number of from 1 to 3.)

In the general formula (1), $R^1$ represents a hydrogen atom or an alkyl group having a carbon number of from 1 to 3. The carbon number of the alkyl group is preferably from 1 to 2, and more preferably 1. The alkyl group having a carbon number of from 1 to 3 for $R^1$ specifically includes a methyl group, an ethyl group, a propyl group, and the like.

$R^1$ is preferably a hydrogen atom or an alkyl group having a carbon number of from 1 to 2, and from the standpoint of avoiding hydrophobization of the vinyl monomer of the present invention, $R^1$ is particularly preferably a hydrogen atom or a methyl group.

In the general formula (1), X influences chain polymerization reactivity and hydrophilic/hydrophobic property of the vinyl monomer of the present invention.

When $R^1$ is a hydrogen atom, X represents a single bond or a divalent organic group having a carbon number of from 2 to 7, which may have a substituent. The carbon number of the organic group is preferably from 2 to 5, and more preferably from 2 to 4.

The divalent organic group which may have a substituent may be any of straight-chain, branched chain or cyclic, and includes, for example, an alkylene group which may have a substituent, an arylene group which may have a substituent, -O-, -C(=O)-, -NH- and groups formed by combination of these groups.

The alkylene group of the alkylene group which may have a substituent includes an ethylene group, a propylene group and the like.

The arylene group of the arylene group which may have a substituent includes an o-phenylene group, a m-phenylene group, a p-phenylene group, and the like.

The substituent of the alkylene group includes, for example, a hydroxyl group; an alkoxy group such as a methoxy group and an ethoxy group; an alkoxycarbonyl group such as a methoxycarbonyl group and an ethoxycarbonyl group; and the like.

The substituent of the arylene group includes, for example, an alkyl group such as a methyl group and an ethyl group.

These substituents may be bonded to any position in the group of the alkylene group and arylene group, and plural number of identical or different substituents may be bonded.

When $R^1$ is a hydrogen atom, X is preferably a single bond or a divalent organic group having a carbon number of from 2 to 5, which may have a substituent, and more preferably a single bond or a divalent organic group having a carbon number of from 2 to 4, which may have a substituent. From the standpoint of imparting the hydrophilicity and chain polymerization reactivity to the vinyl monomer of the present invention, when $R^1$ is a hydrogen atom, X is particularly preferably a single bond, $-C(=O)-$, $-C(=O)-O-(CH_2)_2-$, $-C(=O)-O-CH_2-CH(OH)-CH_2-$, $-C(=O)-NH-(CH_2)_3-$, or the like.

When $R^1$ is an alkyl group having a carbon number of from 1 to 3, X represents a single bond or a divalent organic group having a carbon number of from 1 to 7, which may have a substituent. The carbon number of the organic group is preferably from 1 to 5, and more preferably from 1 to 4.

The divalent organic group which may have a substituent may be any of straight-chain, branched chain or cyclic, and includes, for example, an alkylene group which may have a substituent, an arylene group which may have a substituent, -O-, -C(=O)-, -NH- and groups formed by combination of these groups.

The alkylene group of the alkylene group which may have a substituent includes a methylene group, an ethylene group, a propylene group, and the like.

The arylene group of the arylene group which may have a substituent includes an o-phenylene group, a m-phenylene group, a p-phenylene group, and the like.

The substituents of the alkylene group and arylene group include those described above.

The substituents may be bonded to any position in the group of the alkylene group, arylene group and the like, and plural number of identical or different substituents may be bonded.

When $R^1$ is an alkyl group having a carbon number of from 1 to 3, X is preferably a single bond or a divalent organic group having a carbon number of from 1 to 5, which may have a substituent, and more preferably a single bond or a divalent organic group having a carbon number of from 1 to 4, which may have a substituent. From the standpoint of imparting the hydrophilicity and chain polymerization reactivity to the vinyl monomer of the present invention, when $R^1$ is an alkyl group having a carbon number of from 1 to 3, X is particularly preferably - $C(=O)-$, $-C(=O)-O-(CH_2)_2-$, $-C(=O)-O-CH_2-CH(OH)-CH_2-$, $-C(=O)-NH-(CH_2)_3-$, or the like.

In the general formula (1), n represents 0 or 1.

When n is 0, $R^2$ is a hydrogen atom, an alkyl group having a carbon number of from 1 to 3, an acyl group having a carbon number of from 1 to 2, or an aldehyde group. The carbon number of the alkyl group is preferably from 1 to 2, and more preferably 1. The carbon number of the acyl group is more preferably 2.

The alkyl group having a carbon number of from 1 to 3 for $R^2$ includes a methyl group, an ethyl group and an (iso)propyl group.

The acyl group having a carbon number of from 1 to 2 for $R^2$ includes, for example, an acetyl group.

The aldehyde group for $R^2$ includes, for example, a formyl group.

When n is 0, $R^2$ is preferably a hydrogen atom, an alkyl group having a carbon number of from 1 to 2 or an acyl group having a carbon number of 2, and more preferably a hydrogen atom, a methyl group or an acetyl group.

When n is 1, $R^2$ represents an alkylene group having a carbon number of from 1 to 3. The carbon number of the alkylene group is more preferably from 1 to 2.

The alkylene group having a carbon number of from 1 to 3 for $R^2$ includes a methylene group, an ethylene group and a propylene group.

The vinyl monomer having a diglycolamic-acid-type ligand, represented by the general formula (1) is preferably at least one compound selected from the group consisting of a (meth)acrylic acid compound, a (meth)acrylamide compound, a styrene compound, and a vinylamide(imide) compound.

Specific examples of the vinyl monomer having a diglycolamic-acid-type ligand, represented by the general formula (1) include Compounds (a1) to (a7) represented by formulae below.

[Chem. 3]

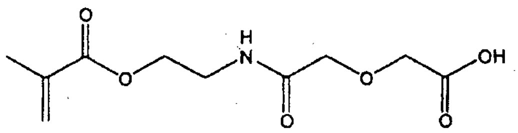

(a1)

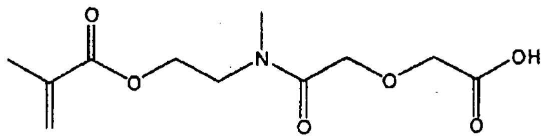

(a2)

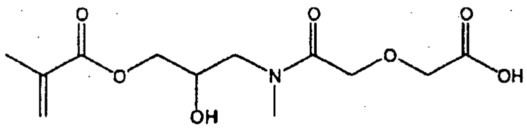

(a3)

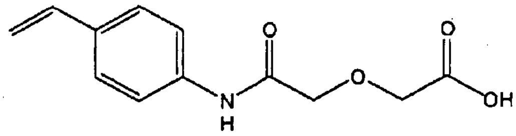

(a4)

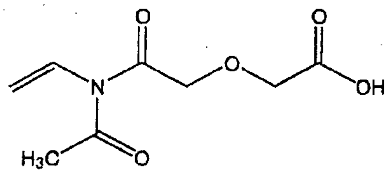

(a5)

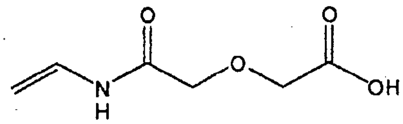

(a6)

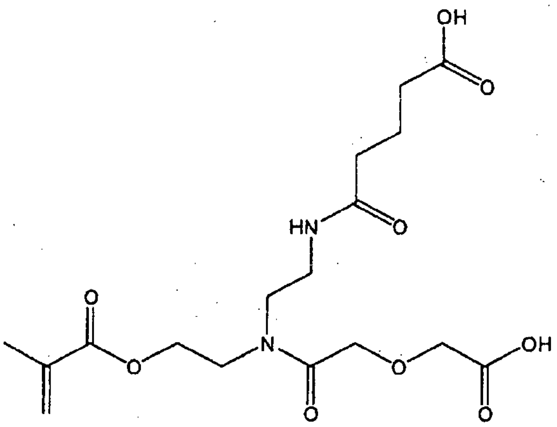

(a7)

Next, a production method of the vinyl monomer of the present invention will be described.

The vinyl monomer of the present invention can be produced by a condensation reaction between a vinyl monomer having at least one or more primary or secondary amines and diglycolic acid. As an activated acid component, diglycolic anhydride which is an acid anhydride is used. Furthermore, by using a symmetric acid anhydride, there is an advantage in that the half of the acid does not react with the amine.

As to the condensation reaction, the case of producing a styrene monomer having DGAA (DGAA styrene monomer) is described as an example.

[Chem. 4]

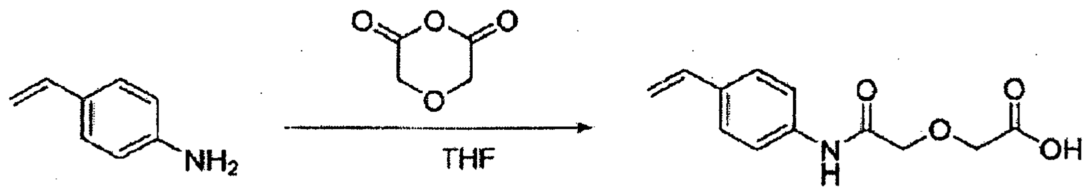


4-Aminostyrene DGAA styrene monomer

For instance, 4-aminostyrene and diglycolic anhydride are allowed to react in an inert solvent, for example, tetrahydrofuran (THF) or dichloromethane under stirring, to obtain a reaction solution.

The resulting reaction solution is filtered, and the filtrate is concentrated and crystallized by adding an organic solvent such as hexane or heptane. The crystals deposited are collected by filtration, washed with heptane and dried, to obtain DGAA styrene monomer.

The vinyl monomer of the present invention is suitably used for the production of an adsorption separation material for a rare earth element.

The adsorption separation material is not particularly limited and includes, for example, a homopolymer and a copolymer each produced by radical polymerization or ionic polymerization, and it is preferred to produce the adsorption separation material by forming a graft chain on a carrier base material of an existing shape by graft polymerization using the vinyl monomer of the present invention.

The material constituting the carrier base material includes, for example, a polyolefin resin, a water-insoluble vinyl alcohol resin, a polyamide resin, and a cellulose resin, and the carrier base material includes a nonwoven fabric, a woven fabric, a non-porous film (sheet), a porous film (sheet), a hollow fiber membrane, yarn, a bead, and the like each formed from the resin described above.

The graft polymerization method for forming the graft chain on the carrier base material is not particularly limited and includes, for example, a chemical graft polymerization method using a heat polymerization initiator such as a peroxide, a graft polymerization method using plasma, a photo-initiated graft polymerization method, and a radiation graft polymerization method. Among them, the radiation graft polymerization method, which does not require an initiator and provides deep penetration depth of treatment, is preferred because the reaction conditions are mild and the graft chain can be formed on the carrier base material without impairing the characteristics of the carrier base material.

The radiation graft polymerization method includes, for example, a method (pre-irradiation method) in which a carrier base material (polymer base material) is irradiated with radiation to generate free radicals (reaction initiation points) and then the carrier base material is brought into contact with a monomer composition to perform graft polymerization using the free radicals as the starting points, and a method (simultaneous irradiation method) in which radiation is irradiated in the state of coexisting a carrier base material and a monomer composition to perform graft polymerization. Since homopolymerization of the monomer is likely to occur simultaneously with the graft polymerization in the simultaneous irradiation method, it is preferred to use the pre-irradiation method.

As the pre-irradiation method, a polymer radical method in which the radiation is irradiated in an inert gas to perform the polymerization may be used or a peroxide method in which the radiation is irradiated in the presence of oxygen to perform the polymerization may be used.

The radiation used for the radiation graft polymerization includes, for example, $\alpha$ ray, $\beta$ ray, $\gamma$ ray, electron beam, accelerated electron beam, and X ray, and may be appropriately determined depending on the material, shape, thickness and the like of the carrier irradiated with the radiation. In general, $\gamma$ ray or electron beam is used in the present invention.

The $\gamma$ ray source includes cobalt, cesium, strontium, and the like. Since the cobalt $\gamma$ ray source has a large penetration depth so that a thickness of the object to be irradiated can be increased in comparison with the case of using electron beam, it can be suitably used in a method in which a long object wound in a roll form is irradiated by a batch process.

As the electron beam source, an accelerator such as Van de Graaff, a linac or a cyclotron is used. Furthermore, the electron beam source has the irradiation dose rate of 300 to 1,000 times higher than that of the $\gamma$ ray source so that short-time irradiation is possible, but since it has a small penetration depth, it can be suitably used in the case where a thin layer object is continuously irradiated by using a conveying device. Since the penetration depth of the accelerated electron beam depends on the acceleration voltage, the acceleration voltage can be appropriately set according to the thickness and the irradiation size of the object to be irradiated.

The irradiation dose of radiation is ordinarily from 10 to 500 kGy, and preferably from 15 to 200 kGy.

The radiation irradiation is usually performed at room temperature (10 to 40°C), and in order to suppress the attenuation of the generated radical, it can be performed in an inert gas (e.g., nitrogen or argon), if desired.

As the solvent used in the solution containing the vinyl monomer of the present invention, use can be made of water, an alcohol such as methanol, dimethylsulfoxide and dimethylformamide which are aprotic polar solvents, an ether, or a mixed solution thereof. The concentration of the vinyl monomer of the present invention in the solution is usually from 10 to 80% by weight, and preferably from 20 to 50% by weight.

The reaction temperature and reaction time in the graft polymerization reaction step are not particularly limited, and may be appropriately set in consideration of a degree of radical generation in the carrier base material, the vinyl monomer used, the desired graft ratio, and the like. It can be conducted usually at from room temperature to 100°C, preferably from room temperature to 80°C, and usually for from 0.5 to 180 minutes, preferably from 1 to 60 minutes.

The weight graft ratio which indicates a degree of graft polymerization in the adsorption separation material can be obtained by the formula shown below.

Weight graft ratio (wt%) = (weight of base material after graft polymerization - weight of base material before graft polymerization)/(weight of base material before graft polymerization) × 100

Furthermore, in the present invention, the vinyl monomer having a diglycolamic-acid-type ligand represented by the general formula (1) can form a homopolymer composed on the vinyl monomer and a copolymer of the vinyl monomer and other monomer.

The monomer which can be copolymerized with the vinyl monomer of the present invention is a hydrophilic vinyl monomer selected from the group consisting of a (meth)acrylic acid compound, a (meth)acrylamide compound, a vinylamide(imide) compound, and a mixture thereof. In the case where the compatibility or affinity of the vinyl monomer to the solution containing rare earth elements is poor, the solvation can be improved by copolymerization with the hydrophilic monomer. Furthermore, in the case where the vinyl monomer itself is hydrophobic or in the case of poor reactivity, it is preferred to use the hydrophilic vinyl monomer as the copolymerization component because the affinity is increased and the reaction proceeds in a chain manner by being induced by the reaction of the copolymerization monomer.

In the present invention, the (co)polymer containing the vinyl monomer having a diglycolamic-acid-type ligand represented by the general formula (1) can be suitably used as the adsorption separation material for a rare earth element.

In the case of using as the adsorption separation material for a rare earth element, the (co)polymer can be used in the form of particles.

As described above, since the vinyl monomer of the present invention has the diglycolamic-acid-type ligand which is effective for adsorption of rare earth element ions, rare earth elements can be selectively separated and recovered from natural mineral resources of low grade or wastes by using the adsorption separation material in which the vinyl monomer of the present invention is introduced and bonded to a carrier base material or the (co)polymer obtained by (co)polymerization of the vinyl monomer of the present invention.

EXAMPLE

The present invention will be described more specifically with reference to Examples, but the present invention should not be construed as being limited to the following examples.

<Method for determining weight graft ratio in adsorption separation material>

The weight graft ratio was obtained by the formula shown below.

Weight graft ratio (wt%) = (weight of base material after graft polymerization - weight of base material before graft polymerization)/(weight of base material before graft polymerization) × 100

<Measurement of bonding amount of DGAA in adsorption separation material>

It was obtained by the formula shown below by taking the change in mass of the base material before and after the DGAA bonding reaction as W.

$$\text{Bonding amount of DGAA (mmol/g)} = (\text{W(mg)/molecular weight of reaction substrate (mg/mmol)/ mass of base material after reaction (g)}$$

(Synthesis Example 1)

<Synthesis of DGAA-vinyl monomer>

[Chem. 5]

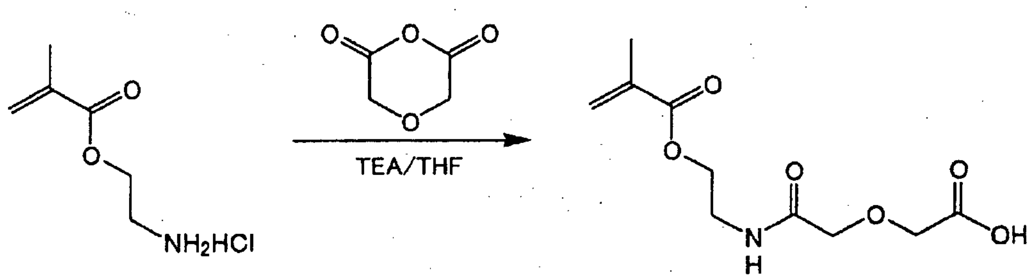


2-Aminoethyl methacrylate hydrochloride

DGAA-methacrylate

In 120 ml of THF was dissolved 16.5 g (0.1 mol) of 2-aminoethyl methacrylate hydrochloride, and under cooling with ice 15.2 g (0.15 mol) of TEA was poured thereto. Thereto was dropwise added 80 ml of a THF solution containing 11.6 g (0.1 mol) of diglycolic anhydride while maintaining at 10°C or less, and the mixture was then back to room temperature and continued to stir overnight (about 12 hours).

The reaction solution was filtered, the filtrate was passed as it was through a silica gel column, THF was flushed as an eluent, and the fraction eluted was concentrated. Toluene was added to the resulting concentrate to crystallize and the crystals deposited were collected by filtration. The resulting crystals were washed with toluene and dried to obtain 11.24 g (yield of 45.7%) of DGAA-methacrylate.

(Preparation Example 1)

<Preparation of adsorption separation material>

A vinylon nonwoven fabric was used as a polymer base material, and a 50 wt% aqueous solution of the DGAA-methacrylate obtained in Synthesis Example 1 was used as a monomer solution. In order to remove dissolved oxygen in the monomer solution, the solution was bubbled for one hour with nitrogen gas.

The polymer base material was irradiated with electron beam of 90 kGy, and the polymer base material irradiated was promptly immersed into the monomer solution. The polymer base material and the monomer solution were allowed to react at 50°C for 15 minutes thereby bonding a graft chain to the polymer base material. Then, after thoroughly washing with pure water, the polymer base material was dried at 40°C for one hour or more to obtain an adsorption separation material.

The weight graft ratio was 136 wt% and the bonding amount of DGAA was 2.35 mmol/g.

(Experimental Example 1)

<Adsorption test of rare earth element>

Each of a chloride salt of dysprosium and a chloride salt of neodymium, as rare earth elements, and chloride salts of copper, iron (III) and zinc, as base metals, was dissolved in distilled water to be 1 mM, and pH was adjusted to

1 or 2 with hydrochloric acid, thereby obtaining each adsorption test aqueous solution.

The adsorption separation material prepared in Preparation Example 1 was added to each of the adsorption test aqueous solutions, and an adsorption test was performed with shaking at about 25°C for one day.

After the adsorption test, the solution was collected and filtered through a membrane filter of 0.20 μm, and a metal ion concentration in the aqueous solution was measured by an ICP emission spectrometer (ICPE-9000 produced by Shimadzu Corp.) to calculate the adsorption amount of metal ion from the mass balance.

The results are shown in FIG. 1.

From the results of FIG. 1, dysprosium and neodymium were adsorbed to the adsorption separation material using the vinyl monomer of the present invention at each pH so that the rare earth elements were able to be selectively adsorbed.

While the present invention has been described in detail and with reference to specific embodiments thereof, it will be apparent to those skilled in the art that various changes and modifications can be made therein without departing from the spirit and scope of the present invention. This application is based on a Japanese patent application filed on September 24, 2014 (Japanese Patent Application No. 2014-194203), and the contents thereof are incorporated herein by reference.

INDUSTRIAL APPLICABILITY

By using the vinyl monomer of the present invention, it becomes easy to bond the vinyl monomer having a diglycolamic-acid-type ligand to a carrier base material, and the resulting adsorption separation material is effective for the separation of rare earth elements.

## Claims

1. A vinyl monomer having a diglycolamic-acid-type ligand, represented by the following general formula (1):

[Chem. 1]

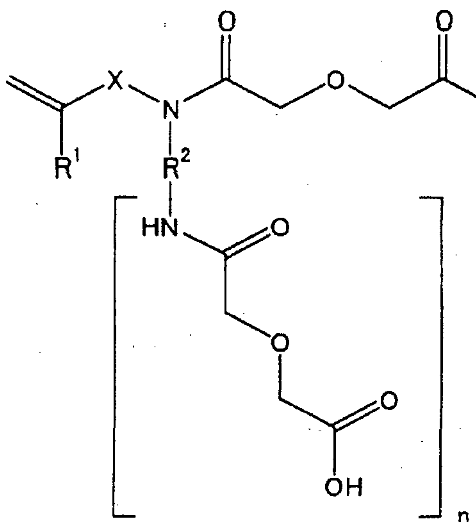

(1)

in the formula (1), $R^1$ represents a hydrogen atom or an alkyl group having a carbon number of from 1 to 3, when $R^1$ is a hydrogen atom, X represents a single bond or a divalent organic group having a carbon number of from 2 to 7, which may have a substituent, and when $R^1$ is an alkyl group having a carbon number of from 1 to 3, X represents a single bond or a divalent organic group having a carbon number of from 1 to 7, which may have a substituent; and n represents 0 or 1, when n is 0, $R^2$ represents a hydrogen atom, an alkyl group having a carbon number of from 1 to 3, an acyl group having a carbon number of from 1 to 2, or an aldehyde group, and when n is 1, $R^2$ represents an alkylene group having a carbon number of from 1 to 3.

2. The vinyl monomer according to Claim 1, which is at least one compound selected from the group consisting of a (meth)acrylic acid compound, a (meth)acrylamide compound, a styrene compound, and a vinylamide(imide) compound.

3. The vinyl monomer according to Claim 1 or 2, which is used for production of an adsorption separation material for a rare earth element.

4. A (co)polymer comprising the vinyl monomer as described in Claim 1 or 2.

5. The (co)polymer according to Claim 4, which is used as an adsorption separation material for a rare earth element.

**Patentansprüche**

1. Vinylmonomer mit einem Ligand vom Diglycolamidsäuretyp, dargestellt durch die folgende allgemeine Formel (1):

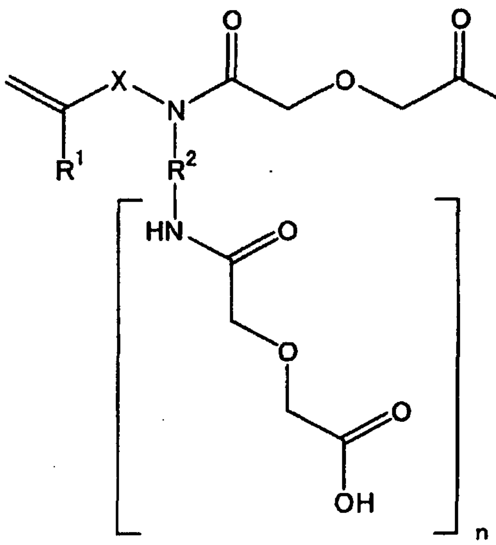

(1)

in der Formel (1) stellt $R^1$ ein Wasserstoffatom oder eine Alkylgruppe mit einer Kohlenstoffzahl von 1 bis 3 dar, wenn $R^1$ ein Wasserstoffatom ist, stellt X eine Einfachbindung oder eine zweiwertige organische Gruppe mit einer Kohlenstoffzahl von 2 bis 7 dar, die einen Substituenten aufweisen kann, und wenn $R^1$ eine Alkylgruppe mit einer Kohlenstoffzahl von 1 bis 3 ist, stellt X eine Einfachbindung oder eine zweiwertige organische Gruppe mit einer Kohlenstoffzahl von 1 bis 7 dar, die einen Substituenten aufweisen kann; und n stellt 0 oder 1 dar, wenn n 0 ist, stellt $R^2$ ein Wasserstoffatom, eine Alkylgruppe mit einer Kohlenstoffzahl von 1 bis 3, eine Acylgruppe mit einer Kohlenstoffzahl von 1 bis 2 oder eine Aldehydgruppe dar, und wenn n 1 ist, stellt $R^2$ eine Alkylengruppe mit einer Kohlenstoffzahl von 1 bis 3 dar.

2. Vinylmonomer nach Anspruch 1, welches mindestens eine Verbindung, ausgewählt aus der Gruppe, bestehend aus einer (Meth)acrylsäureverbindung, einer (Meth)acrylamidverbindung, einer Styrolverbindung und einer Vinylamid(imid)verbindung, ist.

3. Vinylmonomer nach Anspruch 1 oder 2, welche zur Herstellung eines Adsorptionsseparationsmaterials für ein Seltenerdelement verwendet wird.

4. (Co)polymer, umfassend das in Anspruch 1 oder 2 beschriebene Vinylmonomer.

5. (Co)polymer nach Anspruch 4, welches als ein Adsorptionsseparationsmaterial für ein Seltenerdelement verwendet wird.

**Revendications**

1. Monomère de vinyle ayant un ligand de type acide diglycolamique, représenté par la formule générale (1) suivante :

[Produit chimique 1]

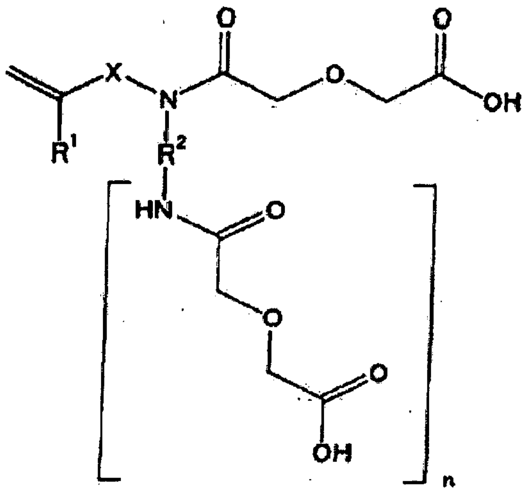

(1)

dans la formule (1), $R^1$ représente un atome d'hydrogène ou un groupe alkyle ayant un nombre d'atomes de carbone allant de 1 à 3, quand $R^1$ est un atome d'hydrogène, X représente une liaison simple ou un groupe organique divalent ayant un nombre d'atomes de carbone allant de 2 à 7, qui peut présenter un substituant, et quand $R^1$ est un groupe alkyle ayant un nombre d'atomes de carbone allant de 1 à 3, X représente une liaison simple ou un groupe organique divalent ayant un nombre d'atomes de carbone allant de 1 à 7, qui peut présenter un substituant ; et n représente 0 ou 1, quand n est 0, $R^2$ représente un atome d'hydrogène, un groupe alkyle ayant un nombre d'atomes de carbone allant de 1 à 3, un groupe acyle ayant un nombre d'atomes de carbone allant de 1 à 2, ou un groupe aldéhyde, et quand n est 1, $R^2$ représente un groupe alkylène ayant un nombre d'atomes de carbone allant de 1 à 3.

**2.** Monomère de vinyle selon la revendication 1, qui est au moins un composé sélectionné parmi le groupe constitué d'un composé d'acide (méth) acrylique, d'un composé (méth)acrylamide, d'un composé styrène, et d'un composé vinylamide(imide).

**3.** Monomère de vinyle selon la revendication 1 ou 2, qui est utilisé pour la production d'un matériau de séparation par adsorption pour un élément de terre rare.

**4.** (Co)polymère comprenant le monomère de vinyle selon la revendication 1 ou 2.

**5.** (Co)polymère selon la revendication 4, qui est utilisé en tant que matériau de séparation par adsorption pour un élément de terre rare.

[FIG. 1]

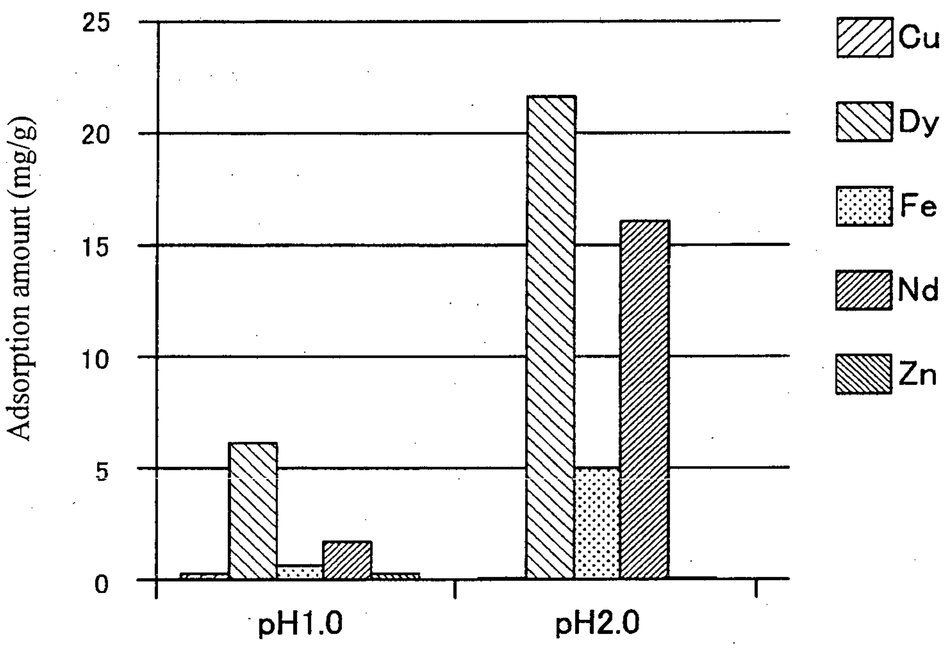

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007327085 A
- JP 2014194203 A


### Non-patent literature cited in the description


- **TSUYOSHI OGATA ; MIKIYA TANAKA.** Separation and recovery of rare earth element ions by novel adsorbent. Rare Earth, The Rare Earth Society of Japan, May 2013, 178-179